(19) Europäisches Patentamt
European Patent Office
Office européen des brevets

(11) **EP 3 951 793 A1**

(12) **EUROPEAN PATENT APPLICATION**

(43) Date of publication:
**09.02.2022 Bulletin 2022/06**

(21) Application number: **20305914.2**

(22) Date of filing: **07.08.2020**

(51) International Patent Classification (IPC):
***G16H 50/20*** (2018.01)

(52) Cooperative Patent Classification (CPC):
**G16H 50/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Imvitro**
**93400 Saint-Ouen (FR)**

(72) Inventors:
• **DELESTRO MATOS, Felipe**
**92240 Malakoff (FR)**
• **BOUSSOMMIER-CALLEJA, Alexandra**
**75013 Paris (FR)**

(74) Representative: **Icosa**
**83 avenue Denfert-Rochereau**
**75014 Paris (FR)**

(54) **DEVICES AND PROCESSES FOR MACHINE LEARNING PREDICTION OF IN-VITRO FERTILIZATION**

(57)  An outcome of an in-vitro fertilization (IVF) of an embryo is predicted by receiving a time sequence of images of the embryo during its IVF development and related clinical feature information, recursively deriving over respective current images of the time sequence, based on at least one machine learning approach, at least one step output associated with one of the current images, from operations applied jointly to that current image and to at least one step output associated with a preceding and/or following image, producing at least one prediction on the fertilization outcome from at least one of the step outputs, and providing the prediction(s). The operations are applied jointly to the clinical feature information in recursively deriving the step output(s) associated with each of the current images.

FIG. 6

**Description**

**TECHNICAL FIELD**

The invention relates to the domain of in-vitro fertilization (IVF), and regards more particularly support to biological decisions based on sequences of embryo images.

**BACKGROUND ART**

Among ways to overcome human infertility, IVF has proved for a number of years an efficient and precious assisted reproductive technology, in which an egg or oocyte removed from a woman's ovaries is fertilized outside the body, in a laboratory.

The egg fertilization results in a zygote, which through embryo culture and cleavage (rapid cell divisions) during 2 to 7 days turns into a morula via compaction, then to a blastocyst via blastulation. The fertilized egg, or embryo, is thereafter implanted into a uterus of either the egg donor or another patient receiving the embryo, i.e. a surrogate mother. From the ninth week after conception, the embryo is then referred to as a fetus.

In spite of significant progress during the latest years, the obtained related pregnancy rate and possibly still more significantly, the live birth rate, remain relatively low, and tend to decrease substantially with the age of the egg donor. For example, in France in 2019, usual birth rates amounted to about 22 % per cycle on average, decreasing to 12 % and 6 % for a female donor aged 38 and 42, respectively.

Also, a possible downside to IVF due to pregnancy health risks is the relatively high probabilities of multiple pregnancy, which may lead to the birth of twins, triplets or more, due to the needed implantation of multiple embryos for increasing the chances of birth success.

In this respect, an appropriate selection of IVF embryos for implantation proves essential, and various predictors of success have been developed over time. Among the useful corresponding tools, time-lapse embryo imaging consists in obtaining a series of pictures (typically thousands) of growing embryos during their development, which enables to study in a non-invasive way morphology and morphokinetic parameters. This avoids potentially damaging effects of removing the embryos from a controlled incubation environment.

In particular, morphokinetics eases morphological selection of embryos by identifying specific developmental events and possibly their timings, such as notably particular cell divisions and/or starts of biological phases including e.g. compaction or blastulation.

The complexity and stakes of the matter, potentially high available volume of data, and persistent uncertainties on key criteria to be considered for successful embryo selection, fostered the rise of dedicated Artificial Intelligence (AI) methods in recent years. Part of them has been able to take account of dynamic aspects of embryo development, which appear to be significantly correlated with IVF outcomes.

Thus, in the article "Automatic grading of human blastocysts from time-lapse imaging" by M. F. Kragh et al., Computers in Biology and Medicine 115 - 103494, 2019, a method based on deep learning is used for automatically grading the morphological appearance of human blastocysts from time-lapse imaging. More precisely, a convolutional neural network (CNN) combined with a recurrent neural network (RNN) are trained so as to incorporate temporal information in jointly predicting inner cell mass (ICM) and trophectoderm (TE) grades.

Also, patent application WO 2020/001914 to Vitrolife discloses establishing machine-learned classifiers for associating images of an embryo belonging to a time series to a likelihood of associations with predetermined developmental events, so as to determine estimated timing for the developmental events. Among various implementations, the use of a bidirectional RNN is suggested so that the classification can be made partly on the basis of information associated with processing of preceding and/or following inputs in the time series.

According to the above documents, the described methods provide helpful information in embryo selection, taking account of an expected significant correlation between the extracted information and the corresponding embryo viability. Anyway, these are not adapted to provide direct predictions on pregnancy or live birth. In fact, the embryo quality may be amount to a mere potential for successful implantation and development in the uterus, rather than to an effective fertilization outcome. Accordingly, whatever the relevant links between the quality of the morphology predictive markers or the timings of the developmental events, on one hand, and a fertilization outcome on the other hand, there remains margin for substantial prediction enhancement.

Patent application WO 2019/113643 to Virtus Innovations proposes a solution for outputting directly a viability score from raw time-lapse video data of a human embryo, that viability score representing a likelihood that the embryo will result in a viable embryo or a viable fetus. This relies on applying at least one 3D artificial neural network to the video data, more precisely disclosed as a 3D CNN. Such a system is presented as extracting not only intra-frame but also inter-frame features, thereby capturing both spatial and temporal features of the embryo. The solution is likewise disclosed (with less implementation details but with complementary results) in the article "Deep learning as a predictive tool for fetal heart pregnancy following time-lapse incubation and blastocyst transfer" by D. Tran et al., Human Reproduction, pp. 1-8, 2018 (publication 2019). According to those documents, a very high performance was obtained where compared with known fetal heart pregnancy outcome, reaching an Area Under the Curve (AUC) as high as 0.78 (patent application) and 0.93 (article) for the Receiver Operating Characteristic (ROC) curve generated by plotting the true positive rate against the false positive rate across all possible thresholding values.

However, it appears that a substantial degree of image compression is required for avoiding excessive computation costs (in terms of power, memory and time), frames of 128x128 pixels being mentioned in the examples. Such a compression may involve losing a lot of information. Also, datasets of 903 and 1281 videos (patent application) and 1835 treatment cycles (article) were processed, which appears relatively small. It is further observed that for the disclosed demanding computations, preprocessing operations are key, notably re-scaling and data augmentation, while only part of them is detailed in the documents. Consequently, it is expected that the performance of the process may not be generally relevant, but instead substantially decrease in some other applications than the considered examples, and may not always be adapted to effective live birth prediction or pregnancy prediction.

Patent application WO 2019/068073 to The Brigham and Women's Hopital, Inc. follows another approach adapted to provide, notably, a likelihood that implantation of the embryo will be successful or will result in a live birth, on the ground of time-lapse images of an embryo. According thereto, a neural network generates values representing the morphology of the embryo from the images, and a downstream expert system evaluates those values to provide an output class representing the above likelihood. There is disclosed more specifically that the neural network may be a CNN or an RNN, the latter allowing to exhibit dynamic temporal behavior (§ 20). In addition, the expert system (which may be a feedforward neural network) may further receive features external to the images, such as biometric parameters of an egg donor, a sperm donor, or a recipient of the embryo (§ 27, 32).

Though this document teaches an attractive solution, thanks to the consideration of the embryo morphology together with the proposed clinical parameters in predicting a fertilization outcome, it may still be penalized by underestimating some important timing aspects of the embryo developments, even though partially reflected in the values representing the morphology where the neural network is an RNN

Consequently, being able to obtain more reliable predictions on embryo fertilization outcomes, while integrating in an enhanced way not only visual factors but also timing information, appears highly desirable.

## SUMMARY

A purpose of the present disclosure is to provide predictions on embryo fertilization outcomes, so as to make possible, in particular, relevant selections of embryos among various batch candidates with a view to implantation.

The present disclosure further pertains notably to a solution based on time-lapse images of embryo developments and on machine learning, enabling to take morphological and morphokinetic embryo development factors into account while directly predicting outcomes of embryo IVF.

## *Preliminary definitions*

In the present disclosure, the term *"embryo"* is used to designate all stages of egg development, from the zygote to the fetus, i.e. including phases sometimes referred to as related to a pre-embryo. The disclosure is directed to humans, but may concern other mammals as well.

The terms "*adapted*" and *"configured*" are used in the present disclosure as broadly encompassing initial configuration, later adaptation or complementation of the present device, or any combination thereof alike, whether effected through material or software means (including firmware).

The term *"processor"* should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

An *"image"* concerns a visual representation of a scene, presently including one or more embryos, at a given point of time. Accordingly, the image may possibly be a frame or a set of frames. Images may be available as parts of a video.

*"Image features"* of one or more images are characteristics extracted from those images, whether having a particular explicit meaning with respect to observed embryos (e.g. size or shape of an embryo) or not (e.g. numerical expressions derived from pixel brightness).

*"Image segmentation"* consists in partitioning a digital image into multiple segments by assigning labels to image pixels, with a view to easier following processing.

The term *"recursive"* takes its usual mathematical or numerical sense of a function being applied within its own definition. Presently, recursion may be applied to successive images respectively corresponding to successive developmental times.

An *"IVF outcome"* may encompass any significant event or piece of information following a transfer to a patient's uterus. This may notably include pregnancy, determined by fetal heart beat (FHB), live birth, and physical characteristics of a newborn such as e.g. weight or height, whether considered alone or in any combination.

*"Clinical feature information"* refers to information related to the embryo IVF and obtained independently of developmental images of the considered embryo(s). This may encompass notably health or history information (e.g. age, number of previous IVF cycles, number of live births, cause of infertility, previous diseases) pertaining to the egg donor, the patient receiving the embryo(s) or the male donor, specificities of eggs collected from one donor (e.g. number of fresh eggs collected, number of rejections), whether taken alone or in any combination.

*"Machine learning (ML)"* designates in a traditional way computer algorithms improving automatically through experience, on the ground of training data enabling to adjust parameters of computer models through gap reductions between expected outputs extracted from the training data and evaluated outputs computed by the computer models.

*"Datasets"* are collections of data used to build an ML mathematical model, so as to make data-driven predictions or decisions. In *"supervised learning"* (i.e. inferring functions from known input-output examples in the form of labelled training data), three types of ML datasets (also designated as ML sets) are typically dedicated to three respective kinds of tasks: *"training",* i.e. fitting the parameters, *"validation",* i.e. tuning ML hyperparameters (which are parameters used to control the learning process), and *"testing",* i.e. checking independently of a training dataset exploited for building a mathematical model that the latter model provides satisfying results.

A *"neural network"* or *"artificial neural network (ANN)"* designates a category of ML comprising nodes (called *neurons*), and connections between neurons modeled by *"weights".* For each neuron, an output is given in function of an input or a set of inputs by an *"activation function".* Neurons are generally organized into multiple *"layers*", so that neurons of one layer connect only to neurons of the immediately preceding and immediately following layers.

A *"Deep Neural Network (DNN)"* is an ANN comprising multiple layers (called *hidden layers)* between an input layer and an output layer.

An *"RNN (Recurrent Neural Network)"* is a class of ANN where connections between neurons form a directed graph along a temporal sequence, which provides a temporal dynamic behavior. Accordingly, an RNN is a particular kind of recursive neural network, defined as an ANN having a same set of weights applied recursively over a differentiable graph-like structure by traversing the structure in topological order. For an RNN, the structure corresponds to a linear chain over a time sequence. The RNN is provided with an internal state (i.e. a memory) keeping track of a previous time step.

A *"bi-directional RNN"* is obtained by concatenating outputs of two RNNs, one processing a time sequence in one direction, and the other processing the time sequence in the opposite direction. This enables to label each element of the sequence based on the past and the future of the element.

An RNN *"with a gated state or gated memory"* is an RNN in which the internal state is under a direct control by the neural network.

A *"Long Short-Term Memory (LSTM)"* is an RNN with a gated state, comprising a cell (unit memory) and three gates regulating the time flow of information into and out of the cell: an input gate (incoming flow), an output gate (outgoing flow) and a forget gate (providing a degree of attenuation of a previous state).

A *"Gate Recurrent Unit (GRU)"* is an RNN with a gated state, comprising a cell (unit memory) and two gates regulating the time flow of information into and out of the cell: an input gate (incoming flow) and a forget gate (providing a degree of attenuation of a previous state).

A *"Convolutional Neural Network (CNN)"* is a class of ANN, and more precisely DNN, in which the hidden layers convolve (cross-correlation) with a multiplication or other dot product, each convolutional neuron processing input data only from a restricted subarea of a previous layer, called a *"receptive field'.*

A *"Generative Adversarial Network (GAN)"* is an ML involving two ANNs contesting with each other (in terms of data distribution), which enables to generate new data from a given training set by learning: one of the ANNs, called a *"generative network",* generates candidates, while the other ANN, called a *"discriminative network",* evaluates them.

The above ML definitions are compliant with their usual meaning, and can be completed with numerous associated features and properties, and definitions of related numerical objects, well known to a person skilled in the ML field.

Additional terms will be defined, specified or commented wherever useful throughout the following description.

### Objects of the disclosure

An object of the present disclosure is notably a device for predicting an outcome of an in-vitro fertilization of an embryo. The device comprises:

- at least one input adapted to receive data representative of a time sequence of images of the embryo during an in-vitro development of the embryo and clinical feature information related to that embryo;
- at least one processor configured for recursively deriving, over respective current images of the time sequence, at least one step output associated with one of the current images, from operations applied jointly to said one of the current images and to at least one step output associated with at least one of a preceding image and a following image in that time sequence, the deriving being based on at least one machine learning approach, and for producing at least one prediction on the fertilization outcome pertaining to that embryo from at least one of the step outputs,
- at least one output adapted to provide the prediction(s).

According to the disclosure, the processor(s) is/are configured for applying those operations also jointly to the clinical feature information in recursively deriving the step output(s) associated with each of the current images.

In the above, a "step output" is an output associated with a process step, which step corresponds to a current image, and accordingly to a given time of the time sequence.

Recursively deriving step outputs from operations applied jointly to the current images and to the step outputs associated with a preceding image and/or a following image in the time sequence amounts to executing a time loop. The latter may define a unidirectional time recursion based on the past and directed to the future, so that an oldest image may provide a starting current image without predecessor and the prediction(s) may be produced from a most recent image via a last associated step output. Conversely, the unidirectional time loop may be based on the future and directed to the past, so that a most recent image may provide a starting current image without predecessor and the prediction(s) may be produced from the oldest image via a last associated step output. Switching from the former implementation to the latter may be merely obtained by reversing the order of the images in the time sequence.

The time loop may alternatively define a bidirectional time recursion, in which the operations are applied jointly to a current image and to step outputs derived from both past and future images in the time sequence - except for the oldest image, which has no preceding image, and for the most recent image, which has no following image.

In some implementations, the recursion may involve at most for each current image an immediately preceding and/or following image. The indirect dependence on more remote images then results from the recursion, since immediately preceding and/or following images themselves depend on one or more of their immediate neighbors. In alternative implementations, the operations may be directly applied jointly to a current image and to step outputs associated with preceding and/or following images up to a given order, whenever existing. For example, the order may be 2 so that wherever possible for a current image, not only the step output(s) associated with the immediately preceding and/or following image in the time sequence should be jointly processed, but also the preceding and/or following image being one image away from the current image.

The machine learning approach may be based on various existing models, as known to a person skilled in the art. In particular, the time loop may be implemented with one or more RNN networks, which may be possibly bidirectional.

The device for predicting compliant with the disclosure makes possible a prediction on the fertilization outcome, thanks to taking into account the clinical feature information. In this respect, it differs significantly from notably the above-cited documents "Automatic grading of human blastocysts from time-lapse imaging", and WO 2020/001914 to Vitrolife, which are targeting intermediary information, such as morphological grades and predetermined developmental events.

It also differs substantially from above-cited documents WO 2019/113643 to Virtus Innovations and "Deep learning as a predictive tool for fetal heart pregnancy following time-lapse incubation and blastocyst transfer", which do not introduce clinical feature information as inputs in their solutions. Though the technologies disclosed therein are designed to provide directly a viability score, it is expected that the relevance of related predictions may be negatively affected by a diversity of clinical backgrounds, for example when applying those methods to egg donors having ages ranging between early and advanced ones. Indeed, while the visualized embryo developments should somehow reflect those clinical backgrounds, relying exclusively thereon for predicting IVF outcomes may induce significant biases, amplified by the high computational resources required for deriving appropriate information.

In some of its disclosed embodiments, i.e. when describing the use of an RNN, WO 2019/068073 to The Brigham and Women's Hopital, Inc. teaches both the exploitation of biometric parameters and of the dynamic temporal behavior of the embryo development in the evaluation of an embryo viability likelihood. However, the present device for predicting still differs significantly from that prior art. Namely, in the latter, the RNN is relied upon in an upstream stage for deriving values representing a morphology of the embryo, and those values are exploited in a downstream stage for obtaining the embryo viability likelihood. By contrast, the present device for predicting fully integrates the consideration of the clinical feature information in a recursive time loop, repeatedly relying on the same clinical feature information at each time step. Consequently, the obtained predictions result from strong interactions between that clinical feature information and the recursive time processing of the images.

In appropriate embodiments, such strong interactions may provide highly relevant predictions, including for various and heterogeneous clinical features. In some implementations, the device for predicting may further offer a good trade-off between required computational resources and prediction reliability. In addition, a global training of the machine learning system may be carried out based on training sets including known IVF outcomes associated with available time sequence images of embryo developments, instead of separate module training as in WO 2019/068073. This may provide strengthened prediction relevance.

The recursive mechanism of the device for predicting may amount to a moving filter analyzing individual images, while receiving as inputs also model parameters obtained from last analyzed image together with the clinical feature information. This creates some sort of internal memory regarding previous predictions. Also, the device for predicting may learn ML parameters (e.g. ANN weights) of clinical features individually, for each "type" of image.

Producing the prediction(s) on the fertilization outcome from one or more of the step outputs may possibly also be based on at least one ML approach. In this case, the ML approach(s) may be the same as for deriving recursively the step output(s) and/or both deriving and producing may be jointly trained.

In some implementations, the device for predicting is configured for preprocessing the data representative of images so as to have the images standardized, as developed below in relation with illustrative embodiments.

In advantageous modes, the processor(s) is/are configured for deriving the step output(s) by applying the operations for said one of the current images in function of a level of consideration of the step output(s) used for those operations. That level of consideration depends on that current image, on the step output(s) used for those operations and on the clinical feature information. That level of consideration is further determined based on a machine learning approach

In this way, the operations themselves depend on the processing of the preceding or following images, making possible an automatic adjustment of the level of consideration of those images when dealing with the current image.

Such modes may create some sort of internal memory units keeping information about particular events to be further used, while the device for predicting may learn what kind of information to store into those memory units from the data themselves.

In more specific implementations, the processor(s) is/are configured for determining a degree of importance of at least one of the images in the time sequence from the level of consideration, and the output(s) is/are adapted to provide that degree of importance.

Obtaining the degree of importance of particular images in the time sequence may make possible a straightforward identification of key time points in the development of the embryo. The degree of importance may e.g. be represented on a graph in function of the time elapsed since insemination. Then, graph peaks likely point to important events for the IVF outcome.

This may notably offer a precious feedback tool for a better grasp of underlying physiological mechanisms of the embryo development, and allow e.g. caregivers or medical staff to focus more particular attention to specific moments with a view to particular care (e.g.

temperature adjustment, composition of a culture medium).

Alternatively or in addition, such a feedback may enable computer engineers or scientists to identify flaws in the automatic processes (such as e.g. dataset selection, digital parameters or software resources) and to provide for appropriate remedies.

That solution may offer significant potential advantages over heat maps obtained with the 3D CNN technology disclosed in WO 2019/113643 to Virtus Innovations, even though such maps are adapted to provide information on time importance together with image area importance. Indeed, that prior art makes use of voxels involving 2-dimension image representation combined with time, within a short time scale (kernel size of 3x3x3). By contrast, the present solution may potentially show the importance of a given time frame in the context of a whole sequence of events.

In particular categories of implementations, the machine learning approach(es) include(s) at least one Recurrent Neural Network, comprising at least one node configured for receiving data related to said one of the current images and to the clinical feature information and for storing data derived from the preceding image(s) or following image(s) and from that clinical feature information. Also, the operations include a time-varying activation applied jointly in that node to the received data and to the stored data.

RNNs prove particularly suited to the present device for predicting, since they rely by nature on a time recursive model.

More precisely, in the above-mentioned embodiments in which the step output(s) is/are derived by applying the operations in function of a level of consideration, at least one of the RNN node(s) has at least one gated state, that level of consideration being given by the gated state(s). The RNN then comprises advantageously a Long Short-Term Memory and/or a Gated Recurrent Unit.

Using an RNN with a gated state proves particularly suited to the presence of that level of consideration, since such a gated state is directed to a self-adjusted control of storage content. As known to a skilled person, LSTMs and GRUs are further appropriate for resolving the vanishing gradient problem (according to which the memory of old event tends to fade away quickly) possibly encountered when training traditional RNNs, also called vanilla RNNs.

Still more precisely, in the embodiments using a level of consideration for determining a degree of importance of one or more of the images in the time sequence, the node(s) having the gated state(s) comprise(s) a forget gate that contributes to the gated state(s) by having a time-varying forget activation function providing the level of consideration, and the processor(s) is/are configured for determining the degree of importance from the forget activation function.

Such implementations may possibly provide a quite efficient and convenient way of deriving the degree of importance of images in a sequence, and consequently of time points, from available parameters. For example, the degree of importance may merely be given by a reciprocal level (i.e. multiplicative inverse) of a forget gate activation function of an LSTM node or a GRU node.

In particular embodiments, the processor(s) is/are configured for extracting image features from data representative of the time sequence of images, and applying the operations to the current images through the image features.

That extraction stage may be useful for reducing the level of complexity load relying on the following time recursive actions, while possibly fully contributing to the prediction process. In particular, the image feature extraction may itself rely on a machine learning approach.

Thus, in some modes, the processor(s) is/are configured for extracting the images features from the images at least by a Convolutional Neural Network, the Convolutional Neural Network(s) being further configured for applying a convolution layer jointly to the image features and to the clinical feature information.

Such an implementation appears particularly suited, since CNNs are quite adapted to image processing, the connectivity pattern between neurons resembling the organization of an animal visual cortex. Also, the presence of the jointly applied convolution layer may enable to combine in an efficient way the image information and clinical feature information, so as to feed dedicated time recursive kernels with already synthetic data.

In advantageous implementations, the prediction(s) on said fertilization outcome regards pregnancy, live birth, and/or physical attributes of a newborn, such as e.g. weight or height.

Different versions of a model compliant with the device for predicting may be trained and exploited for different outcome predictions, such as the ones of the above advantageous implementations.

The prediction(s) may take the form of one or more prediction score(s). Alternatively or in combination, the prediction(s) may be expressed as outcome probabilities.

The predictions may be expressed as viability scores, supplying embryologists with a ranking of multiple embryos belonging to a same batch regarding their probability of success. This may thus be an aid for the selection of the most suitable embryo, or possibly embryos, the rest of the procedure for embryo transfer continuing as usual for the clinic.

In advantageous modes, the clinical feature information regards at least one of:

- features pertaining to an egg donor, including an age, a number of total collected fresh eggs, a number of total created embryos, a number of total rejected embryos, a cause of infertility, a number of total previous in-vitro fertilization cycles, a total number of live births, a cause of woman infertility,

- features pertaining to a patient receiving said embryo, including an age, a body mass index, a treatment regime, a hormonal profile, a past diagnosis of a condition, an endometrium thickness,
- features pertaining to a sperm donor, including a past diagnosis,
- features pertaining to a history of said embryo, including an egg age, an egg maturation status, a type of used stimulation, an elective single embryo transfer, a fertilization method.

Another object of the disclosure is a method for predicting an outcome of an in-vitro fertilization of an embryo, that method comprising:

- receiving data representative of a time sequence of images of the embryo during an in-vitro development of the embryo and clinical feature information related to the embryo;
- recursively deriving, over respective current images of the time sequence, at least one step output associated with one of the current images, from operations applied jointly to said one of the current images and to at least one step output associated with at least one of a preceding image and a following image in the time sequence, the deriving being based on at least one machine learning approach, and producing at least one prediction on the fertilization outcome pertaining to the embryo from at least one of the step outputs,
- providing the prediction(s).

According to the disclosure, the method comprises: applying the operations also jointly to the clinical feature information in recursively deriving the step output(s) associated with each of the current images.

The method for predicting is advantageously executed by a device for predicting according to any of its embodiments.

The disclosure further regards a device for training a machine learning system configured for predicting an outcome of an in-vitro fertilization of an embryo. That device comprises:

- at least one input adapted to receive at least one set of data representative of time sequences comprising images of embryos during in-vitro developments of the embryos and of known information relevant to fertilization outcomes for the respective embryos;
- at least one processor configured for iteratively over the time sequences:

  recursively deriving, over respective current images of any of the time sequences, at least one step output associated with one of the current images, from operations applied jointly to said one of the current images and to at least one step output associated with at least one of a preceding image and a following image in said any of the time sequences, the deriving relying on training parameters of the machine learning system,

producing evaluated information relevant to the fertilization outcomes for the respective embryos from at least one of the step outputs,

comparing the evaluated information with the known information, and modifying the training parameters so as to reduce discrepancies between the evaluated information and the known information,

stopping iteratively proceeding with the deriving, producing, comparing and modifying when at least one stop criterion is met,

- at least one output adapted to provide the modified training parameters.

According to the disclosure:

- the input(s) is/are adapted to receive the set(s) including clinical feature information related to the embryos,
- the processor(s) is/are configured for applying the operations also jointly to the clinical feature information in recursively deriving the step output(s) associated with each of the current images,
- the known information comprises data on the fertilization outcomes for the respective embryos and the evaluated information comprises predictions on the fertilization outcomes for the respective embryos.

The device for training is advantageously adapted to a device for predicting according to any of its embodiments.

Comparing the evaluated information with the known information and modifying the training parameters should not be understood as necessarily meaning that two distinct and successive steps need to be executed. By contrast, they may be combined in a joint operation, e.g. through a gradient descent optimization method.

The comments made above about the device for predicting regarding the images, the related representative data and their preprocessing stand likewise about the device for training. Also, functionalities of the device for training regarding the nature and form of the input image data and of the output information are advantageously identical to, similar to, or at least consistent with, the functionalities of the device for predicting (e.g. image or video compression format, type of processed data). Notably, a preprocessing of images providing a same standardization form for both devices may be executed for sake of efficiency and/or reliability.

A further object of the disclosure is a method for training a machine learning system configured for predicting an outcome of an in-vitro fertilization of an embryo, the method comprising:

- receiving at least one set of data representative of time sequences comprising images of embryos during in-vitro developments of the embryos and of known information relevant to fertilization outcomes for the respective embryos;
- iteratively over the time sequences:

    recursively deriving, over respective current images of any of the time sequences, at least one step output associated with one of the current images, from operations applied jointly to said one of the current images and to at least one step output associated with at least one of a preceding image and a following image in said any of the time sequences, the deriving relying on training parameters of the machine learning system,

    producing evaluated information relevant to the fertilization outcomes for the respective embryos from at least one of the step outputs,

    comparing the evaluated information with the known information, and modifying the training parameters so as to reduce discrepancies between the evaluated information and the known information,

    stopping iteratively proceeding with the deriving, producing, comparing and modifying when at least one stop criterion is met,

- providing the modified training parameters.

Accordingly to the disclosure, the method comprises:

- receiving the set(s) including clinical feature information related to the embryos,
- applying the operations also jointly to the clinical feature information in recursively deriving the step output(s) associated with each of the current images,

the known information comprising data on the fertilization outcomes for the respective embryos and the evaluated information comprising predictions on the fertilization outcomes for the respective embryos.

The method for training is advantageously executed by a device for training according to any of its execution modes.

The device for predicting and/or the device for training may be implemented in various ways with proper associated user interface, whether together or separately, notably as a standalone software installed in a computer, a cloud-based service or an Application Programming Interface (API).

In addition, the disclosure relates to a computer program comprising software code adapted to perform a method for predicting or a method for training compliant with any of the above execution modes when the program is executed by a processor.

The present disclosure further pertains to a non-transitory program storage device, readable by a computer, tangibly embodying a program of instructions executable by the computer to perform a method for predicting or a method for training, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

## LIST OF FIGURES

The present disclosure will be better understood, and other specific features and advantages will emerge upon reading the following description of particular and non-restrictive illustrative embodiments, the description making reference to the annexed drawings wherein:

**Figure 1** is a block diagram representing schematically a particular mode of a device for predicting an outcome of an embryo IVF, compliant with the present disclosure;

**Figure 2** represents a timeline showing the importance of individual frames in a predicted IFV outcome;

**Figure 3** represents a heat map representing regions of an image of an embryo that gave most important features in a predicted IFV outcome;

**Figure 4** illustrates a particular implementation of the device for predicting of figure 1, using a combined CNN and RNN processing;

**Figure 5** represents the implementation of figure 4 in a condensed form, while pointing out its time recursive construction;

**Figure 6** provides more details on a CNN block in the implementation of figures 4 and 5;

**Figure 7** illustrates an LSTM cell, as used in a specific embodiment of the implementation of figures 4 to 6;

**Figure 8** is a flow chart showing successive steps executed with the device for predicting of figure 1;

**Figure 9** shows an embryo selection process involving an IVF treatment, exploiting the device for predicting of figure 1;

**Figure 10** is a block diagram representing schematically a particular mode of a device for ML training, compliant with the present disclosure and adapted to the device for predicting of figure 1;

**Figure 11** is a flow chart showing successive steps executed with the device for training of figure 10;

**Figure 12** diagrammatically shows an apparatus integrating the functions of the device for predicting of figure 1 and of the device for training of figure 10.

On the figures, the drawings are not to scale, and identical or similar elements are designated by the same references.

## ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its spirit and scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

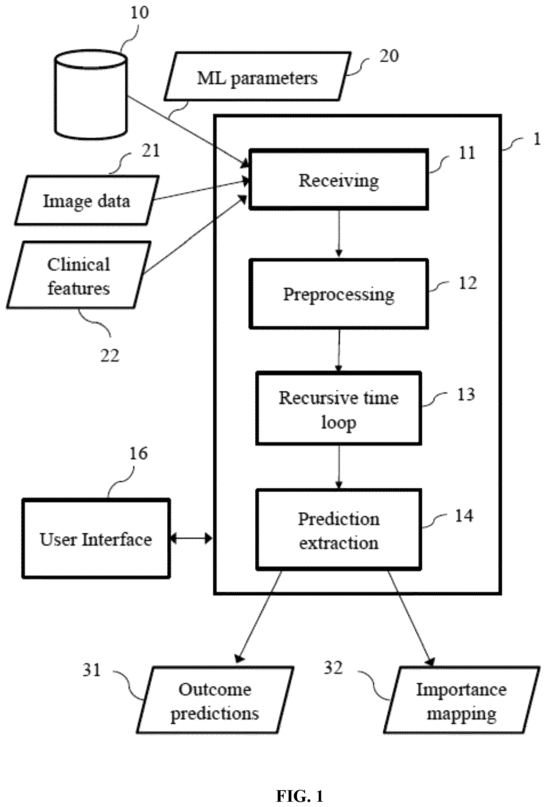

The present disclosure will be described in reference to a particular functional embodiment of a device 1 for predicting an outcome of an embryo IVF, as illustrated on **Figure 1.**

The device 1 is adapted to produce such outcome predictions 31 from data 21 representative of a time sequence of images of one or more embryos during IVF (hereinafter "image data") and from clinical features 22 related to that embryo or those embryos. The outcome predictions 31 may comprise one prediction score and/or one probability per embryo, the score or probability pertaining to a chance of successful IVF outcome, understood as pregnancy or live birth. They may instead or in addition regard physical characteristics of the newborn, such as weight or height.

In advantageous embodiments, the device for predicting 1 is further adapted to produce importance mapping information 32, giving a feedback on the relative importance of image areas (space mapping) and/or of time frames (time mapping) in inducing the outcome predictions 31. That mapping information 32 may be visual, and take e.g. the form of a heat map for the space mapping, and of a timeline for the time mapping.

The image data 21 may be derived from videos of embryo development, obtained by time-lapse embryo imaging (TLI) techniques. They may be derived from pictures or videos obtained with any microscopy TLI apparatus, such as e.g. EmbryoScope® (Vitrolife), Geri® (Merck) or MIRI® (ESCO). They may be periodically time-spaced. In alternative embodiments, the time lapses between at least some of consecutive images may be irregular.

The images may be grayscale images or color images. The image data 21 may include numerical data, such as digital data. Those data may include individual image data in a compressed form, as well known to a person skilled in image compression, such as e.g. in compliance with JPEG (for Joint Photographic Experts Group), JPEG 2000 or HEIF (for High Efficiency Image File Format) standard. They may instead, or in addition, include video data in a compressed form, notably relying on motion compensation, such as e.g. in compliance with MPEG-4 Part 2 (MPEG standing for Moving Picture Experts Group), H.264 (also called AVC, standing for Advanced Video Coding), H.265 (also called HEVC, standing for High Efficiency Video Coding), or H.266 (also called VVC, standing for Versatile Video Coding) standard.

For a given prediction running, the image data 21 may be derived from a unique TLI apparatus used for acquiring a video or a sequence of images pertaining to a given embryo or batch of embryos. Alternatively, images may be issued from two or more TLI apparatus, or even from two or more different kinds of TLI apparatus.

The image data 21 may e.g. comprise between 300 and 2000 frames, captured during 2 to 6 days, typically 5 days.

The clinical features 22 may comprise information collected independently of the image data 21, such as information about an egg donor, a sperm donor or a patient ready to receive the post-IVF embryos, or information about pre-conditions of the embryos (as a zygote or an egg) before its placement into a culture medium.

A particularly interesting set of the clinical features 22, ordered in consideration of a potential importance, is as follows: 1/ Patient age at treatment, 2/ Total fresh eggs collected, 3/ Total embryos created, 4/ Cause of egg donor infertility, 5/ Total rejected embryos, 6/ Total number of previous IVF cycles, 7/ Total number of live births, 8/ Stimulation used, 9/ Cause of male infertility, 10/ Elective single embryo transfer.

Different clinical features may possibly be adopted together as inputs while being respectively allotted with distinct consideration weights, in order to reflect an importance order of consideration in IVF outcome.

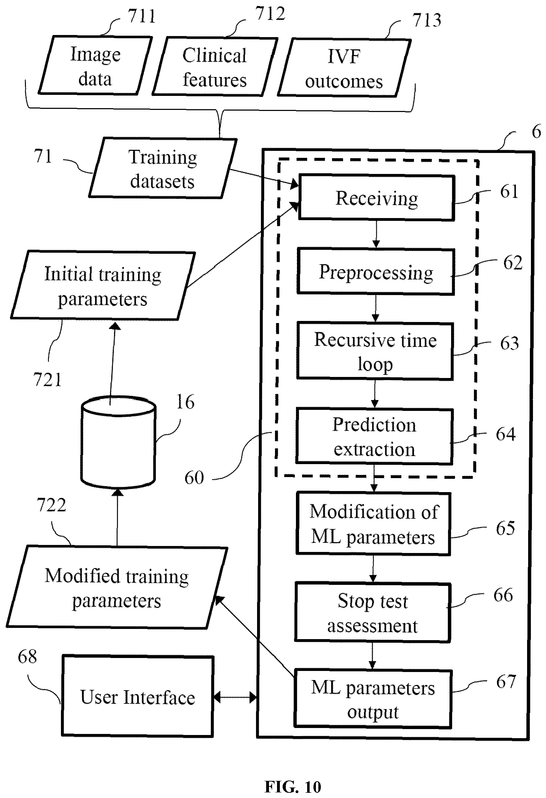

The device 1 for predicting is associated with a device 6 for training suited to setting model parameters of the device 1 in a proper way, represented on **Figure 10,** which will be subsequently described.

Though the presently described devices 1 and 6 are versatile and provided with several functions that can be carried out alternatively or in any cumulative way, other implementations within the scope of the present disclosure include devices having only parts of the present functionalities.

Each of the devices 1 and 6 is advantageously an apparatus, or a physical part of an apparatus, designed, configured and/or adapted for performing the mentioned functions and produce the mentioned effects or results. In alternative implementations, any of the device 1 and the device 6 is embodied as a set of apparatus or physical parts of apparatus, whether grouped in a same machine or in different, possibly remote, machines. The device 1 and/or the device 6 may e.g. have functions distributed over a cloud infrastructure and be available to users as a cloud-based service, or have remote functions accessible through an API.

The device 1 for predicting and the device 6 for training may be integrated in a same apparatus or set of apparatus, and intended to same users. In other implementations, the structure of device 6 may be completely independent of the structure of device 1, and may be provided for other users. For example, the device 1 may have a parameterized model available to operators for IVF outcome predictions, wholly set from previous training effected upstream by other players with the device 6.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1 or of the device 6.

The device 1 comprises a module 11 for receiving the image data 21 and the clinical features 22, as well as ML (machine learning) parameters 20 stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In advantageous embodiments, the ML parameters 20 have been previously generated by a system including the device 6 for training. Alternatively, the ML parameters 20 are received from a communication network.

The device 1 further comprises optionally a module 12 for preprocessing the received image data 21 and possibly the clinical features 22. The module 12 may notably be adapted to standardize the received image data 21 for sake of efficient and reliable processing. It may transform the image data 21, e.g. by image or video decompression. It may extract features from the image data 21. According to various configurations, the module 12 is adapted to execute only part or all of the above functions, in any possible combination, in any manner suited to the following processing stage.

In particular embodiments, the module 12 is configured for decompressing the images and/or videos, according to proper decompression standard(s), and the images are dealt with in a decompressed form. In other embodiments, the device 1 is configured for operating at least partly on compression-resulting image or video data, e.g. on DCT (Discrete Cosine Transform) values.

Data on more (possibly many more) images and/or image frames than the processed ones may be received, the presently considered images resulting from selection operations by the module 12. For example, an image may comprise multiple frames having respective focal planes (e.g. 9 focal planes), and only three of them may be kept for processing (e.g. with a center focal and two peripheral focals). Also, a time selection of available pictures may be applied beforehand, for example by keeping one image out of four, so that if e.g. one picture was shot every 15 minutes, one image per hour is exploited in the device for predicting. Alternatively or in combination, original pictures may be pre-combined into available images, for example by averaging frames of a same image and/or by averaging successive images within predetermined durations.

In some embodiments, a single embryo is visible on the processed images. While multiple embryos may be cultured together in a same incubator, typically constituting a batch of embryos originating from a same egg donor, pictures automatically taken by a camera may show the various embryos developing together. However, the module 12 may execute an appropriate image processing to generate image data exclusively directed to a single one of those embryos, for example by including appropriate cropping and/or pixel deletion.

In alternative embodiments, multiple embryos are visible on the processed images. The device 1 is then adapted to provide a prediction on the fertilization outcome pertaining to at least one of those embryos. For example, a specific embryo among a batch of embryos may be recognized over the time sequence through a dedicated pre-coloring by the module 12, effected through an automated processing familiar to a person skilled in the art (relying e.g. on edge detection and possibly using two or more complementary available frame angles of the pictures).

Also, multiple embryos may be assessed jointly by the device 1, so that respective predictions are provided for the considered embryos. This may be achieved e.g. by assigning with the module 12 a different label to each of the latter. In some implementations, the device 1 is then adapted to process separately each of the identified embryos, the processing for the various embryos being executed either successively, or in parallel by respective identical processing units. In other implementations, the device 1 is adapted to process globally the considered embryos visible on the images for providing outcome predictions. Though such global operations may be quite more computationally demanding and potentially introduce more imprecisions, they may in return offer a more straightforward and quicker processing, subject to appropriate computer resources.

In advantageous modes, the module 12 is configured for preprocessing the image data 21 so as to have the images standardized. This may enhance the efficiency of the downstream processing by the device 1. Such a standardization may be particularly useful when exploited images originate from different sources, including possibly different imaging systems.

The standardization is advantageously applied to the image data 21 and to image sequences of training datasets (e.g. by the device 6) in a similar way. Notably, the device 1 may deal with image data coming from a given sort of source, while its parameters result from a training based on image data obtained with different types of sources. Thanks to standardization, differences between sources may then be neutralized or minimized. This may make the device 1 more efficient and reliable.

In the learning phase, the device 6 may use datasets comprising image data that originate from multiple TLI apparatus and/or kinds of TLI apparatus, as well as from different clinics. Integrating same image standardization in the devices 1 and 6 allows to get consistent outputs when running the device 1.

In some modes, the module 12 comprises a pipeline of standardization steps, which may e.g. include at least some of the following operations: removal of identification signs (banners, time stamps, logos...), detection and removal of a culture well and creation of a neutral background, detection and centralization of a target embryo in a frame, adjustment of magnification to a common standard, normalization of illumination and of image sharpness to a common standard. In advantageous implementations, the pipeline is specifically built and finetuned for each kind of microscope.

In particular standardization modes, the module 12 comprises one or more Generative Adversarial Network (GAN), adapted to include in a training dataset, images obtained by a given specific imaging system, e.g. EmbryoScope® (Vitrolife), or more precisely by a given imaging apparatus. In this way, images originating from other TLI apparatus or types of TLI apparatus may be transformed so that they look like the reference ones. Such an automatic process may be advantageous in avoiding multiple successive operations and/or manual efforts, while providing standardized images relevant thanks to the self-validation property of a GAN network.

In addition, preprocessing the images may include a circular segmentation (i.e. segmentation applied to a circular region), typically enabling to differentiate image background and embryo, and between distinct embryos. That segmentation may be exploited for appropriate circular cropping (i.e. cropping applied to a circular region).

The device 1 also comprises a module 13 for recursive time looping, devoted to dealing with the preprocessed data obtained from the receiving module 12. It may be observed that the operations by the modules 11, 12 and 13 are not necessarily successive in time, and may overlap, proceed in parallel or alternate, in any appropriate manner. For example, new image data may be progressively received over time and preprocessed, while the module 13 is dealing with the previously obtained image data. In alternative examples, a batch of image data 21 corresponding to a complete time sequence range may be fully received and preprocessed before it is submitted to the module 13.

The recursive time loop of the module 13 may rely on an iterative mechanism applied to successive images (i.e. to corresponding image data) of a time sequence, therefore to successive points of time, in which an output obtained for a current image associated with a given time (hereinafter a "step output") is feeding a processing of a following image as an input. The module 13 may then proceed with such successive steps in a chronological order. In other implementations, the module 13 may deal with the successive steps in an anti-chronological order from the future to the past, by retrieving step outputs from a later time when executing a current step. In still other implementations, the module 13 may combine both approaches according to a bidirectional time approach, so that step inputs for a current image are coming from the past and from the future.

The module 13 may process a considered current image by retrieving data relating to that current image as well as a step output relating to a previously processed image, or step outputs relating to multiple previously processed images. As mentioned above, the previous processing may refer to the past, but instead or in addition, to the future as well. However, this may not apply to each current image, in particular since an appropriate related step output may not always be available. In particular, an initial image may have no previously processed image in the time sequence, so that the module 13 may process it without any step output from a previously processed image. For example, the initial image is the first one in the time sequence with a processing in the chronological order, or the last one in the time sequence with a processing in the anti-chronological order. An initial image may instead have one or more previously processed image in the time sequence, but less than provided for a complete case. For example, the initial image is the first one or the last one in the time sequence, and a bidirectional time approach is followed.

The module 13 is thus adapted to apply jointly operations to a current image of the time sequence and to one or more step outputs respectively associated with previously processed images (before and/or after in the time sequence), so as to produce itself a step output for the current image. In addition, the module 13 is adapted to retrieve and take account of the clinical features 22, which are therefore made a full inside part of the recursive loop.

The operations carried out by the module 13 are based on an ML approach, and thus on previously determined proper parameters.

One or more RNNs (Recurrent Neural Networks) may be particularly suited to such a time recursive loop, due to their recurrent nature. This is developed through particular examples below.

Anyway, other ML implementations may be used instead of or in combination with RNNs. Namely, the time recursion of the device for predicting as defined above may be carried out with multiple other ML solutions.

For example, the ML approach may comprise a binary classifier adapted to provide a positive or negative fertilization outcome prediction, such as a Support Vector Machine (SVM). The ML approach may comprise one or more Classification Trees, in which predictions are associated with leaves. An ML ensemble learning based on Decision Trees may also be exploited, such as Random Forests. The ML approach may comprise one or more probabilistic graphical models, such as dynamic Bayesian networks.

The module 13 may be adapted to extract image features from the received preprocessed data by an ML approach. As seen above, the preprocessing module 12 may already have itself a function of image feature extraction, so that deciding whether the latter should be considered as part of the recursive time loop or rather of the upstream preprocessing stage would be of little practical interest. Anyway, considering the image feature extraction by an ML approach as part of the recursive time loop makes full sense in view of the upstream training. Indeed, such a training may advantageously be based on the functionalities of the device 1 (e.g. by using the device 6), and jointly deal with ML parameters associated with the image feature extraction together with the step output operations. Then, in the overall training operations, image feature extraction is a full part of the recursive time loop. For sake of consistency with the training, the same may be considered for the device 1 for predicting.

For example, the module 13 may include one or more CNNs for extracting image features from the image data 21. As known to a skilled person, such deep neural networks are particularly suited to visual imagery analysis.

More precisely, the module 13 may comprise a U-Net network (as e.g. described in the article "U-Net: Convolutional Networks for Biomedical Image Segmentation", by O. Ronneberger et al., MICCAI, Vol. 9351, pp. 234-241, 2015), which is directed to image segmentation.

In other modes, the module 13 may proceed with an extraction of image features by another ANN technology, such as a Multilayer Perceptron (MLP), or another ML architecture. It may notably use tools such as SVM, Decision Trees or Independent Component Analysis (ICA, separating a multivariate signal into additive subcomponents, and involving unsupervised feature learning).

In other modes, the image feature extraction relies on a segmentation approach. The latter may notably comprise a watershed processing (which finds lines running along the tops of brightness ridges). The segmentation approach may instead, or in combination, comprise histogram-based processing (based on pixel intensities), or an edge-detection processing.

In advantageous implementations, the module 13 is adapted to combine for a current image the image features extracted from the image data 21 with the clinical features 22, so as to form combined data to be processed with the appropriate operations.

The module 13 may be provided with the additional functionality of deriving the step output associated with a current image by applying the operations for that current image in function of a level of consideration of the step output(s) used for those operations. That level of consideration is determined based on an ML approach, and depends on that current image, on the step output(s) used for those operations and on the clinical features 22.

The level of consideration associated with the processing of a current image may e.g. amount to allotted a weight to an input coming from a previously processed image (i.e. to the associated step output), so that the weight itself is determined jointly by that input, the current image and the clinical features 22.

An LSTM (Long Short-Term Memory) or a GRU (Gated Recurrent Unit) may be particularly suited to such a weighted consideration of the incoming step outputs, due to the presence of gated states, as will be developed below.

The device 1 further comprises a module 14 for extracting the outcome predictions 31 from the step outputs executed by the module 13 and for outputting those predictions 31. This may correspond to the step output associated with the lastly processed image, such as the last image of the time sequence for a treatment in the chronological order or the first image of the time sequence for a treatment in the anti-chronological order. The module 14 may instead extract the outcome predictions from two or more step outputs associated with respective images, such as the first and the last images of the time sequence for a bidirectional time treatment.

In addition, the module 14 may execute specific operations on the obtained step output(s). For example, those operations may include a convolution, a clustering, a statistical process or an ML method based e.g. on a Decision Tree, an SVM or a Bayesian network. In case an ML approach is used by the module 14, it may advantageously rely on a common training with the module 13, so that proper model parameters are determined jointly.

The module 14 may optionally be configured for extracting and outputting the importance mapping information 32, which may notably be done from available outputs and/or from ML parameters set during the operations of the module 13.

In particular, the module 14 may determine a degree of importance of at least one of the images in the time sequence from the above level of consideration, when available. Namely, the levels of consideration associated with respective images then flexibly reflect their importance in the prediction process.

As will be detailed below, the presence of an LSTM or a GRU makes such an extraction particularly convenient, notably based on a forget gate.

In other modes, the module 14 is configured for determining the degree of importance of time frames by a SHAP explainer (SHapley Additive explanations). Such a tool is disclosed e.g. in the article "A Unified Approach to Interpreting Model Predictions" by S. M. Lundberg et S.-I. Lee, 31st Conf. on Neural Information Processing Systems, Long Beach, USA, 2017.

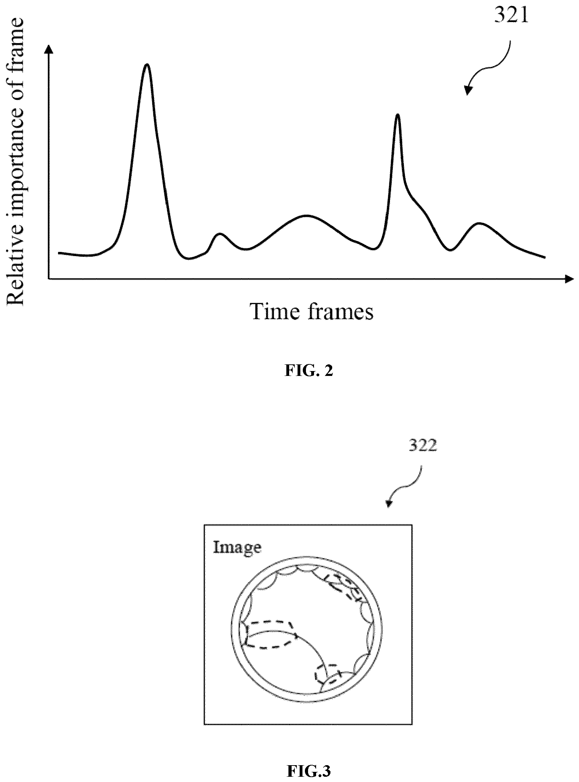

The module 14 may more precisely provide a time importance mapping, insofar as sufficient information is available for the time sequence. This may take the visual form of a graph showing the degree of importance in function of time, as visible on the timeline 321 of **Figure 2.**

Also, in some embodiments, the module 14 is configured for determining a degree of importance of at least one area in at least one of the images in the outcome predictions 31. This may take the form of a heat map or saliency map, providing a visualization of different ranges of degree of importance (space importance mapping), e.g. by different colors or brightness intensities. An example of such a heat map 322 of an embryo is represented on **Figure 3,** where dotted lines represent regions of the image that were more important for the classification. Such embodiments can provide space-related feedback, alternative to, or advantageously complementary to, the above time-related feedback.

Determining the degree of importance of the image area(s) may be obtained by an occlusion process, as known to a skilled person and described e.g. in WO 2019/113643 to Virtus Innovations. Accordingly, specific position areas of an image are respectively occluded (which may be done by replacing image pixels with black pixels), and outputs (i.e. predictions) respectively associated with the occluded areas are yielded. Comparing those outputs with the output of the complete image is then exploited for determining the contributions of the respective image areas.

In other modes involving a CNN network in the time recursive loop, the processor(s) is/are configured for determining the degree of importance of the image area(s) and for possibly generating a feedback heat map by a Grad-CAM method. The latter is described e.g. in the article "Grad-CAM Visual Explanations from Deep Networks via Gradient-based Localization" by R. R. Selvaraju et al., IEEE ICCV, Venice, pp. 618-626, 2017.

The device 1 is interacting with a user interface 16, via which information can be entered and retrieved by a user. The user interface 16 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

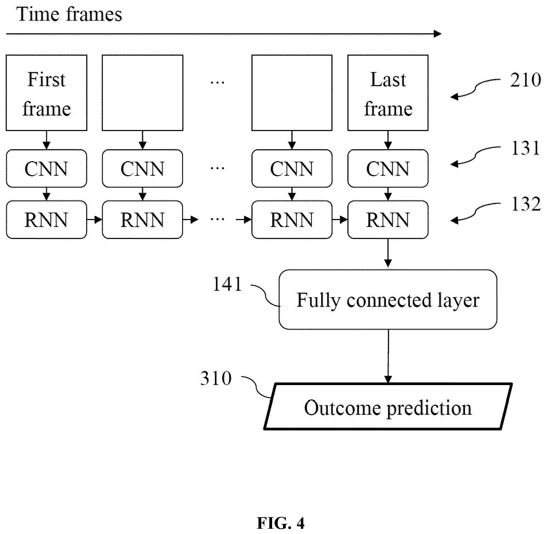

A particularly efficient implementation of the device 1 for predicting will be developed in relation with **Figure 4,** showing an unfolded representation of a recursive time loop. In this example, frames 210 are processed in their chronological order from a first frame to a last frame, and the module 13 relies on CNN blocks 131 for feature extraction and RNN blocks 132 for time recursion. More precisely, each of the RNN blocks 132 is adapted to receive from one of the CNNs 131, features extracted from one of the frames 210, as well as an output from the previous upstream RNN block except for the first RNN block in the chain.

Also, the module 14 in this example comprises a fully connected layer 141, arranged downstream of the last RNN block so as to collect its output. The outcome prediction 310 can be derived from this layer 141.

In a learning phase, that model is able to learn the temporal evolution of data, as information is passed to new blocks while the system analyses the whole dataset.

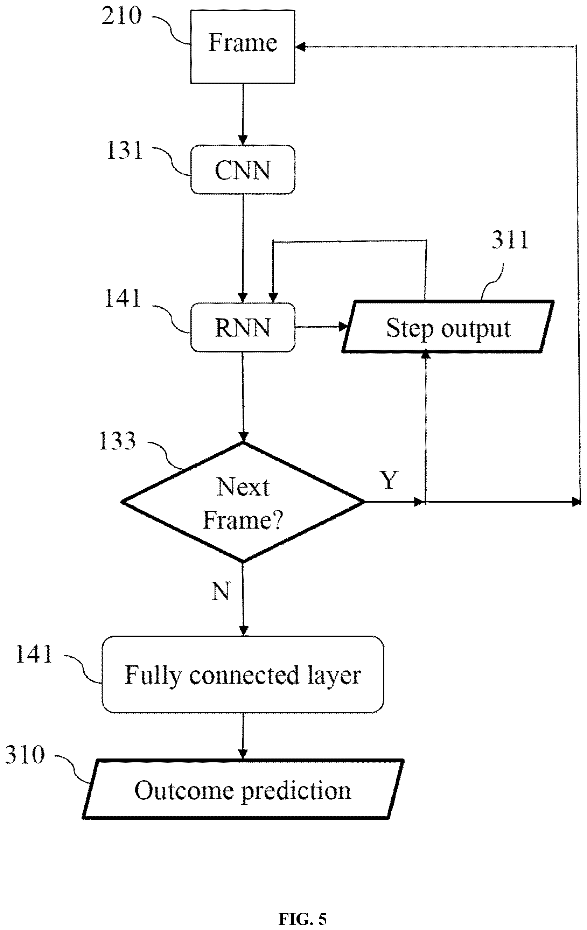

For sake of better understanding, the same architecture represented on **Figure 4** in an unfolded form is shown on **Figure 5** in a condensed form so as to point out the time recursion. A single CNN block 131 receiving image data from frames 210 is then connected downstream to a single RNN block 132, which has a time loop controlled by a test unit 133. As long as the last of the frames 210 is not dealt with, the test unit 133 triggers the processing of a next frame by the CNN block 131 and the feeding of the RNN block 132 by its own output 311 (i.e. its step output), so that the RNN block 132 receives both outputs from the CNN block 131 and from the RNN block 132 for executing its operations. In practice, the RNN block 132 is provided with a memory or internal state, enabling it to keep track of the previous step.

When the test unit indicates that the last of the frames 210 has been processed, the last output 311 is provided to the fully connected layer 141 for deriving the outcome predictions 310.

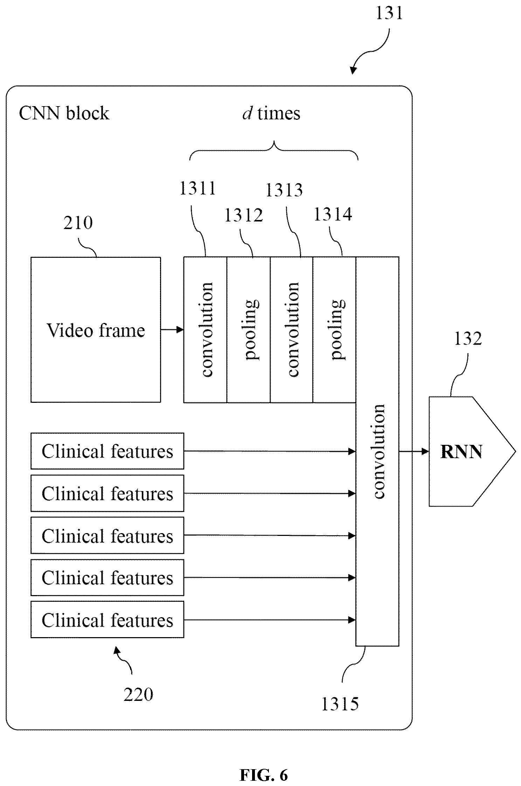

An important aspect of the CNN block 131, shown on **Figure 6,** is that it integrates the clinical features 22. This stands for the running of the device 1 as well as for the related training, e.g. with the device 6. More precisely, the CNN block 131 comprises alternating convolution layers 1311, 1313 and pooling layers 1312, 1314, dedicated to receiving image data from the video frames 210 and to proceeding with image feature extraction. The convolution layers 1311, 1313 are directed to convolution operations, using e.g. as an activation function a ReLU layer (for Rectified Linear Unit, which removes negative values from an activation map by setting them to zero). The pooling layers 1312, 1314 are directed to non-linear down-sampling, and may e.g. carry out max pooling (i.e. partitioning the input image into non-overlapping rectangle regions and outputting a maximum value for each of those regions).

The CNN block 131 also includes a downstream convolutional layer 1315, receiving the clinical features 22 together with the output of the last alternating convolutional layers 1311, 1312, 1313, 1314, and generating from a combined convolutional processing an output provided to the RNN block 132.

In the learning phase, this allows the system to learn relationships between the image data 21 and the clinical features 22.

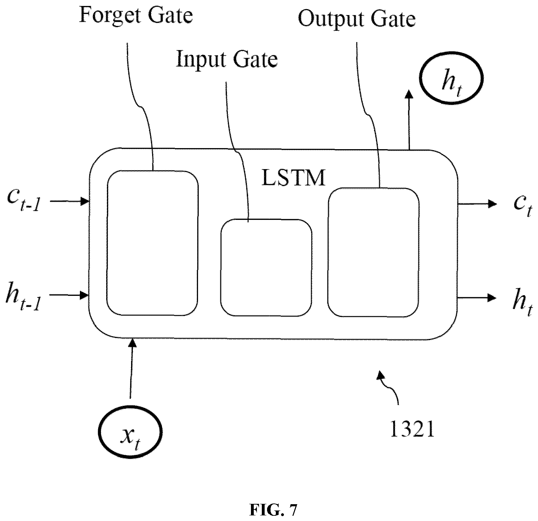

In an advantageous implementation of the above architecture, the RNN block 132 consists in an LSTM block 1321, as represented on **Figure 7.** This notably enables to overcome the vanishing gradient problem possibly arising when training vanilla (i.e. traditional) RNNs. Another benefit of LSTM is its relative insensitivity to gap length in the time recursion.

The LSTM block 1321 at time $t$ is provided with two entries from a previous LSTM block: a cell state vector at time $(t-1)$ noted $c_{t-1}$ and a hidden state vector at time $(t-1)$ noted $h_{t-1}$, considered as a representative output of the LSTM block 1321 at the previous step. It is also provided with two outputs $c_t$ (cell state vector at time $t$)

and $h_t$ (hidden state vector at time $t$) to a next similar LSTM block. As explained above for the RNN blocks 132, the previous and next LSTM blocks in an unfolded form representation of the recurrence may be in fact the same LSTM block 1321. The LSTM block 1321 is also provided with an entry dedicated to an input vector at time $t$, noted $x_t$, and with an off-LSTM output of the hidden state $h_t$.

The LSTM block 1321 comprises three successive gates:

- a Forget Gate adapted to produce from $c_{t-1}$, $h_{t-1}$ and $x_t$ a forget gate's activation vector $f_t$,

- an Input Gate adapted to produce from $h_{t-1}$ and $x_t$ an input gate's activation vector $i_t$, and

- an Output Gate adapted to produce from $h_{t-1}$ and $x_t$ an output gate's activation vector $o_t$.

The cell state vector $c_t$ and the hidden state vector $h_t$ are then obtained from the following equations also involving a cell input activation vector $\check{c}_t$ at time $t$, with "o" designating the Hadamard product (i.e. element-wise):

$$f_t = \sigma_g \left( W_f x_t + U_f h_{t-1} + b_f \right)$$

$$i_t = \sigma_g \left( W_i x_t + U_i h_{t-1} + b_i \right)$$

$$o_t = \sigma_g \left( W_o x_t + U_o h_{t-1} + b_o \right)$$

$$\check{c}_t = \sigma_h \left( W_c x_t + U_c h_{t-1} + b_c \right)$$

$$c_t = f_t \circ c_{t-1} + i_t \circ \check{c}_t$$

$$h_t = o_t \circ \sigma_h \left( c_t \right)$$

with $W$ and $U$ standing for weight matrices and $b$ for bias vector parameters to be learned during training (the subscripts $f$, $i$, $o$ and $c$ corresponding to the concerned vectors), and $\sigma_g$ and $\sigma_h$ standing respectively for the logistic sigmoid function and the hyperbolic tangent function. The initial values are further chosen as:

$$c_0 = 0, \; h_0 = 0$$

An additional specific advantage of the LSTM block 1321 concerns the extraction by the module 14 of a time importance mapping. Namely, in advantageous implementations, the degree of importance of one of the frames 210 is computed from the forget gate of the LSTM block 1321. This degree may e.g. be given by the inverse level of the forget gate activation, i.e. $1 / \|f_t\|$.

In another advantageous implementation of the above architecture, the RNN block 132 consists in a GRU block (not represented). It differs from the LSTM block 1321 by the absence of an output gate, as known to a skilled person.

The GRU block can provide advantages similar to an LSTM with respect to vanilla RNNs (notably in overcoming the vanishing gradient problem). Also, it comprises a forget gate that may be exploited by the module 14 for extracting a degree of importance of time frames, like with an LSTM.

The selection between LSTM and GRU may be made on various considerations. Notably, a GRU is technically simpler than LSTM and usually needs less processing power, while LSTM may outperform GRU is some situations.

Instead of an LSTM or a GRU, another RNN having a gated state may be exploited in the device 1. Alternatively, a vanilla RNN may be used. As mentioned above, instead of processing the frames 210 in the chronological order, the module 13 may process them in the reverse time direction. The module 13 may also exploit a bi-directional RNN. The module 13 may further be built on one or more RNNs, possibly including an LSTM or a GRU, provided with attention mechanisms (enabling, at a given time recursion step, to access and draw from all previously processed steps). In addition, the CNN block may be replaced with another ANN, insofar as the clinical features 22 are dealt with as inputs together with the image data 21. The CNN block may further be replaced with a non-ANN ML architecture.

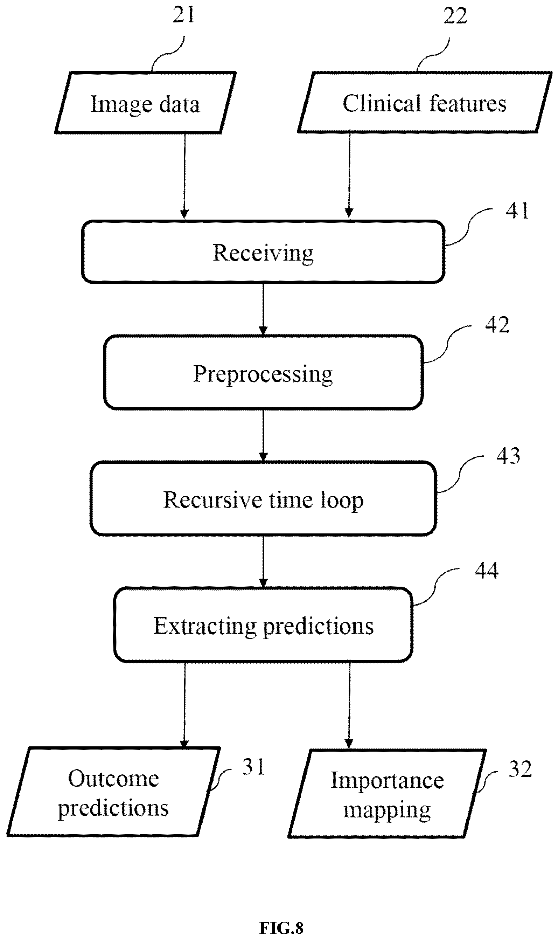

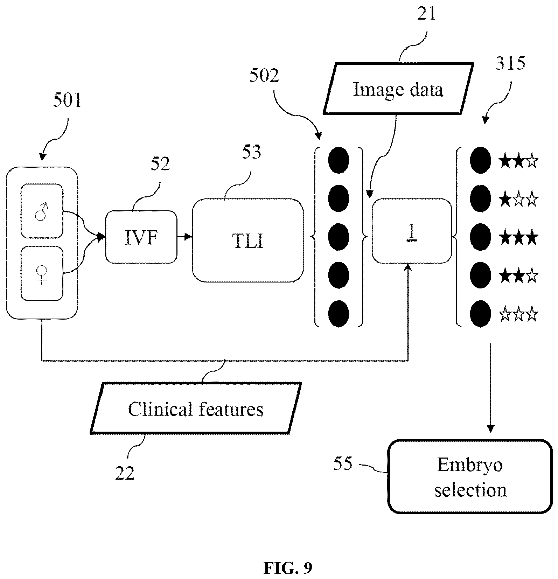

In operation, a process as described below may for example be executed, in relation with **Figure 8** and **Figure 9.** In a situation of infertility issues, male and female gametes 501 are fused so as to produce zygotes, which are subject to an IVF treatment 52 leading to time-lapse imaging 53 of a resulting embryo culture. This results in videos of embryos 502, which are submitted as image data 21 to the device 1 for predicting. In addition, clinical features 22 in relation with the gametes 501 are provided to the device 1. From the image data 21 and the clinical features 22, the device 1 automatically outputs prediction viability scores 315 for the respective embryos 502. Those scores 315 are relied upon by embryologists, at least as aid, for a selection 55 of the embryo or embryos having the highest chance of positive outcome with a view to transfer. The rest of the procedure for embryo transfer may continue as usual for a clinic. In addition, importance mapping information 32 is optionally available, which offers besides the scores 315 a visual feedback on which video segments and/or which areas of the images were most relevant for the decision of the system.

The viability scores 315 pertaining to a batch of embryos may further be used to make decisions on what embryos should be frozen or discarded.

Embryologists may further use average viability scores of several batches of embryos to assess the efficacy of a clinic. It should then be advantageously verified whether changes of methodology, e.g. new equipment or different culture mediums, have an impact on an overall viability.

For a given embryo, an embryologist may use the associated prediction for making a better decision whether a transfer should be made or not. Also, based on a probability of success, the embryologist may give to involved persons (egg and/or sperm donors and/or patient receiving the embryo) more precise feedback on the chances of having a successful outcome.

In its automatic actions, the device 1 may for example execute the following process **(Figure 8):**

-    receiving the image data 21 and the clinical features 22 (step 41),

-    preprocessing the image data 21 and possibly the clinical features 22 with a view to more efficient and/or reliable processing (step 42),

-    carrying out a recursive time loop on the image data 21 after their preprocessing, while iteratively integrating the clinical features 22 as inputs with the image data 21 (step 43),

-    extracting and providing the outcome predictions 31, and possibly also the importance mapping information 32 (step 44).

More information will be given hereinafter on the device 6 for training. The device 6 may be incorporated in a same apparatus as the device 1 for predicting and share most functionalities of the device 1. More generally, the device 1 and the device 6 may have common processors configured for executing part of the functionalities of device 1, whether localized or distributed. In alternative implementations, the device 1 and the device 6 may be completely disconnected, and possibly exploited by distinct players (for example a service provider for the device 6 and clinic staff for the device 1).

In advantageous embodiments, the device 6 shares a same architecture with the device 1, as well as a same data preprocessing. For example, the recursive time loop and the preliminary image standardization are identical. This is however not necessary, and the device 6 may have a different architecture to some extent, insofar as it is able to provide model parameters suited to proper predictions by the device 1.

Also, the training parameters 20 exploited by the device 1 may originate from other sources than the device 6.

As represented on **Figure 10,** the device 6 is adapted to retrieve initial training parameters 721 defining an initial algorithmic model within the frame of an ML architecture, from one or more local or remote database(s) 60. The latter can take the form of storage resources available from any kind of appropriate storage means, which can be notably a RAM or an EEPROM such as a Flash memory, possibly within an SSD. In advantageous embodiments, the initial training parameters 721 have been previously generated by the device 6 for training. Alternatively, the initial training parameters 721 are received from a communication network.

The device 6 is configured for deriving modified training parameters 722 defining a modified algorithmic model on the ground of the entry training parameters 721, and for transmitting those parameters 722 or recording them, e.g. in the database(s) 60. This is achieved by using training datasets 71 received by the device 6, enabling to progressively fit the training parameters so that an associated algorithmic model becomes more relevant to those training datasets 71 (supervised learning).

More precisely, the training datasets 71 comprise inputs including image data 711 and clinical features 712, and outputs including known IVF outcomes 713 respectively corresponding to the inputs. Also, the device 6 is configured for iteratively producing evaluated IVF outcomes from the input image data 711 and clinical features 712, on the ground of training parameters defining a current algorithmic model, and for modifying those training parameters so as to reduce the gaps between the evaluated IVF outcomes and the known IVF outcomes 713.

The modified training parameters 722 can themselves be taken as initial training parameters for subsequent training operations.

Though the training aspects of the device 6 are presently developed, it can be observed that the device 6 may be applied likewise to validation sets and testing sets.

The device 6 for training comprises a running stage 60 including a module 61 for receiving the training datasets 71 and the training parameters 721, a module 62 for preprocessing the image data 711 and optionally the clinical features 712 of the training datasets 71, a module 63 for recursive time looping and a module 64 for extracting the evaluated IVF outcomes from results obtained from the module 63 and for outputting them. Those modules 61, 62, 63 and 64 will not be detailed here, since their description is similar to the description above of the respective modules 11, 12, 13 and 14 of the device 1 for predicting. The ML-based models in modules 62, 63 and/or 64 are set by current values of the training parameters.

In advantageous implementations, the modules 61, 62, 63 and 64 of the device 6 are at least partly identical to the modules 11, 12, 13 and 14 of the device 1. In particular, a same image standardization may be applied (modules 12 and 62), a same ML architecture may be used for the recursive time loop (modules 13 and 63) and/or same methods may be carried out for deriving image features from the image data (modules 12 and 62, or modules 13 and 63) and for extracting the outcome predictions 31 and the evaluated IVF outcomes (modules 14 and 64). In particular, the running stage 60 may be a common entity of the device 1 and the device 6. By "same ML architecture" and "same methods", it is meant subject to the possible exploitation of distinct training parameters.

The device 6 further comprises a module 65 for comparing the evaluated IVF outcomes with the known IVF outcomes 713 and for modifying the current training parameters with a view to reducing the gap between them. The terms "comparisons" and "modifications" are used for sake of simplified expression, while in practice, both operations may be closely combined in synthesis actions, e.g. through a gradient descent.

The device 6 also includes a module 66 for stopping the modifications of the training parameters based on the datasets 71. That module 66 may provide for stopping the related iterations when the gaps between the evaluated IVF outcomes and the known IVF outcomes 713 become smaller than a predefined threshold. It may instead or cumulatively provide an end to those iterations when a predefined maximum number of iterations is reached.

In a minimal core training version, the device 6 is configured for proceeding with training exclusively directed to the recursive time loop. In other modes, the device 6 comprises additional ML stages, which may be notably for extracting image features from the image data 711 and/or for extracting the evaluated IVF outcomes from outputs of the module 63. Then, the device 6 may also be configured for training those additional ML stages. In particular embodiments, the device 6 is configured for jointly training some or all of the additional ML stages together with the recursive time loop of the module 63.

In implementations involving RNNs in the recursive time loop, the device 6 may be adapted to execute backpropagation through time (BPTT). According to that training method, a gradient-based technique is applied to a cost with respect to all network parameters once the RNN is unfolded in time, as known to a skilled person. Alternatively or in combination, a Connectionist Temporal Classification (CTC) network may be exploited for training. The latter may prove particularly suited in case of irregular inter-images durations, and/or when the ML system comprises one or more LSTMs. Also, a stochastic gradient descent may be used.

For example, with the above described implementation involving the CNN blocks 131 and the RNN blocks 132, a Residual Neural Network (ResNet) may be used, with transfer learning from a pre-trained model that used e.g. the visual database known as the ImageNet dataset. The related parameters may be e.g.:

```
num_layers: 18
epochs: 10
optimizer: sdg (Stochastic Gradient Descent)
momentum: 0.9
weight_decay: 0.0001
learning_rate: 0.005
```

```
mini_batch_size: 64
image_shape: 3,224,224
```

The device 6 also comprises a module 67 for providing the modified training parameters 722 resulting from the training operations by the device 6.

It is further interacting with a user interface 68, for the description of which the reader may refer to the user interface 20 associated with the device 1.

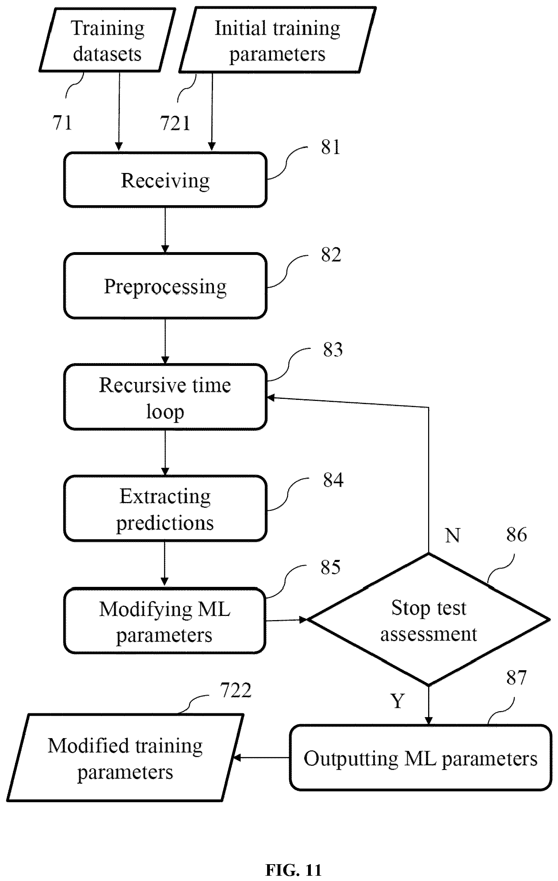

The device 6 may for example execute the following process **(Figure 11):**

- receiving the initial training parameters 721 and one or more of the training datasets 71, or part thereof, including the inputs comprising the image data 711 and the clinical features 712 (step 81) and the outputs comprising the known IFV outcomes 713,

- preprocessing the image data 711 and possibly the clinical features 712 with a view to more efficient and/or reliable processing (step 82),

- carrying out a recursive time loop on the image data 711 after their preprocessing, while iteratively integrating the clinical features 712 as inputs with the image data 711 (step 83),

- comparing the evaluated outcomes with the known outcomes 713 and modifying the current training parameters accordingly (step 85),

- checking the stop criteria (step 86),

- if the stop criteria are not met, iteratively coming back to step 83 with the amended values of the current training parameters, and reiterating the operations,

- if the stop criteria are met, keeping the currently modified training parameters as the modified training parameters 722 and providing them (step 87).

In variant execution modes, the modified training parameters 722 may be distinct from the currently modified training parameters when the stop criteria are met, and be computed from those currently modified training parameters together with one or more previously obtained parameter values (e.g. by averaging).

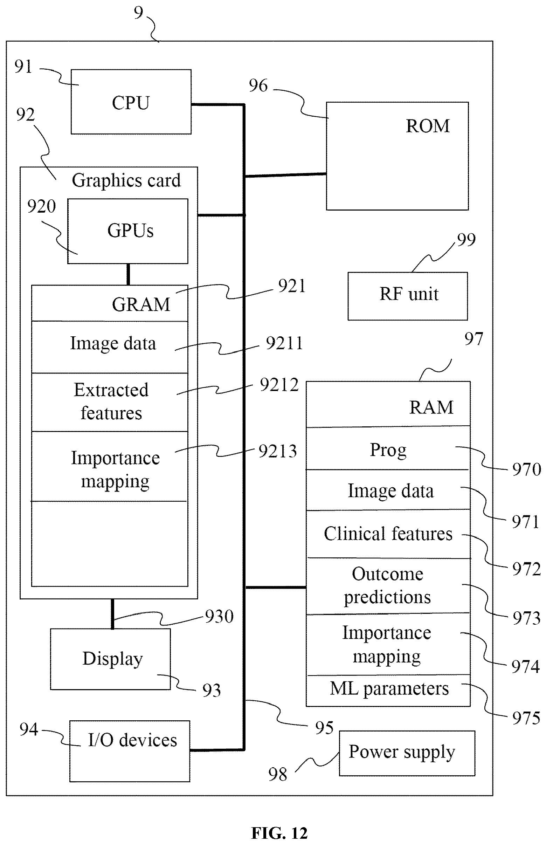

A particular apparatus 9, visible on **Figure 12,** is embodying the device 1 as well as the device 6 described above. It corresponds for example to a workstation, a laptop, a tablet, a smartphone, or a head-mounted display (HMD).

That apparatus 9 is suited to IVF outcome predictions and to related ML training. It comprises the following elements, connected to each other by a bus 95 of addresses and data that also transports a clock signal:

- a microprocessor 91 (or CPU) ;

- a graphics card 92 comprising several Graphical Processing Units (or GPUs) 920 and a Graphical Random Access Memory (GRAM) 921; the GPUs are quite suited to image processing by e.g. the CNN blocks 131, due to their highly parallel structure;

- a non-volatile memory of ROM type 96;

- a RAM 97;

- one or several I/O (Input/Output) devices 94 such as for example a keyboard, a mouse, a trackball, a webcam; other modes for introduction of commands such as for example vocal recognition are also possible;

- a power source 98 ; and

- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card, for example for obtaining heat maps related to the importance mapping information 32. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a videoprojector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

The random access memory 97 comprises notably:

- in a register 970, the operating program of the microprocessor 91;

- in a register 971, the image data 21;

- in a register 972, the clinical features 22;

- in a register 973, the outcome predictions 31;

- in a register 974, the importance mapping information 32;

- in a register 975, the current ML parameters.

Algorithms implementing the steps of the method specific to the present disclosure and described above are stored in the memory GRAM 921. When switched on and once the parameters 971 to 975 are loaded into the RAM 97, the graphic processors 920 of graphics card 92 load appropriate information and parameters into the GRAM 921 and execute the instructions of algorithms in the form of microprograms.

The random access memory GRAM 921 comprises notably:

- in a register 9211, the image data 21;

- in a register 9212, the features extracted from the image data 21;

- in a register 9213, the importance mapping information 32.

As will be understood by a skilled person, the presence of the graphics card 92 is not mandatory, and can be replaced with entire CPU processing and/or simpler visualization implementations.

In variant modes, the apparatus 9 may include only the functionalities of the device 1, and not the learning capacities of the device 6. In addition, the device 1 and/or the device 6 may be implemented differently than a standalone software, and an apparatus or set of apparatus comprising only parts of the apparatus 9 may be exploited through an API call or via a cloud interface.

## Claims

1. A device (1) for predicting an outcome of an in-vitro fertilization of an embryo, said device comprising:

   - at least one input adapted to receive data representative of a time sequence of images (21; 210) of said embryo during an in-vitro development of said embryo and clinical feature information (22; 220) related to said embryo;

- at least one processor configured for recursively deriving, over respective current images of said time sequence, at least one step output (311) associated with one of said current images (210), from operations applied jointly to said one of said current images and to at least one step output associated with at least one of a preceding image and a following image in said time sequence, said deriving being based on at least one machine learning approach, and for producing at least one prediction (31; 310) on said fertilization outcome pertaining to said embryo from at least one of said step outputs,
- at least one output adapted to provide said at least one prediction, **characterized in that**:
- said at least one processor is configured for applying said operations also jointly to said clinical feature information (22; 220) in recursively deriving said at least one step output associated with each of said current images.

2. The device (1) for predicting according to claim **1, characterized in that** said at least one processor is configured for deriving said at least one step output (311) by applying said operations for said one of said current images (210) in function of a level of consideration of said at least one step output used for said operations, said level of consideration depending on said one of said current images, said at least one step output used for said operations and said clinical feature information, said level of consideration being determined based on a machine learning approach.

3. The device (1) for predicting according to claim **2, characterized in that** said at least one processor is configured for determining a degree of importance (32; 321) of at least one of said images in said time sequence from said level of consideration, and said at least one output is adapted to provide said degree of importance.

4. The device (1) for predicting according to any of the preceding claims, **characterized in that** said at least one machine learning approach includes at least one Recurrent Neural Network (132), comprising at least one node configured for receiving data related to said one of said current images (210) and to said clinical feature information (22; 220) and for storing data derived from said at least one of a preceding image and a following image and from said clinical feature information, said operations including a time-varying activation applied jointly in said node to said received data and to said stored data.

5. The device (1) for predicting according to claims **2** and **4, characterized in that** at least one of said at least one node has at least one gated state, said level of consideration being given by said at least one gated state, said Recurrent Neural Network (132) comprising advantageously at least one of a Long Short-Term Memory (1321) and a Gated Recurrent Unit.

6. The device (1) for predicting according to claims **3** and **5, characterized in that** said at least one node having said at least one gated state comprises a forget gate contributing to said at least one gated state by having a time-varying forget activation function ($f_t$) providing said level of consideration, and said at least one processor is configured for determining said degree of importance (32; 321) from said forget activation function.

7. The device (1) for predicting according to any of the preceding claims, **characterized in that** said at least one processor is configured for:

- extracting image features from said data representative of said time sequence of images (21; 210), and
- applying said operations to said current images through said image features.

8. The device (1) for predicting according to claim **7, characterized in that** said at least one processor is configured for extracting said images features at least by a Convolutional Neural Network (131), said Convolutional Neural Network being further configured for applying a convolution layer (1315) jointly to said image features and to said clinical feature information (22; 220).

9. The device (1) for predicting according to any of the preceding claims, **characterized in that** said at least one prediction (31; 310) on said fertilization outcome regards at least one of pregnancy, live birth, and physical attributes of a newborn.

10. The device (1) for predicting according to any of the preceding claims, **characterized in that** said clinical feature information (22; 220) regards at least one of:

- features pertaining to an egg donor, including an age, a number of total collected fresh eggs, a number of total created embryos, a number of total rejected embryos, a cause of infertility, a number of total previous in-vitro fertilization cycles, a total number of live births, a cause of woman infertility,
- features pertaining to a patient receiving said embryo, including an age, a body mass index, a treatment regime, a hormonal profile, a past diagnosis of a condition, an endometrium thickness,
- features pertaining to a sperm donor, including a past diagnosis,

- features pertaining to a history of said embryo, including an egg age, an egg maturation status, a type of used stimulation, an elective single embryo transfer, a fertilization method.

11. A method for predicting an outcome of an in-vitro fertilization of an embryo, said method comprising:

   - receiving (41) data representative of a time sequence of images (21; 210) of said embryo during an in-vitro development of said embryo and clinical feature information (22; 220) related to said embryo;
   - recursively deriving (43), over respective current images of said time sequence, at least one step output (311) associated with one of said current images (210), from operations applied jointly to said one of said current images and to at least one step output associated with at least one of a preceding image and a following image in said time sequence, said deriving being based on at least one machine learning approach, and producing (44) at least one prediction (31; 310) on said fertilization outcome pertaining to said embryo from at least one of said step outputs,
   - providing (44) said at least one prediction,

   **characterized in that** said method comprises:

   - applying said operations also jointly to said clinical feature information (22; 220) in recursively deriving said at least one step output associated with each of said current images,
   said method for predicting being advantageously executed by a device (1) for predicting according to any of claims **1** to **10.**

12. A device (6) for training a machine learning system configured for predicting an outcome of an in-vitro fertilization of an embryo, said device comprising:

   - at least one input adapted to receive at least one set (71) of data representative of time sequences comprising images (711) of embryos during in-vitro developments of said embryos and of known information (713) relevant to fertilization outcomes for said respective embryos;
   - at least one processor configured for iteratively over said time sequences:

      recursively deriving, over respective current images of any of said time sequences, at least one step output (311) associated with one of said current images (210), from operations applied jointly to said one of said current images and to at least one step output associated with at least one of a preceding image and a following image in said any of said time sequences, said deriving relying on training parameters of said machine learning system,
      producing evaluated information relevant to said fertilization outcomes for said respective embryos from at least one of said step outputs,
      comparing said evaluated information with said known information (713), and modifying said training parameters so as to reduce discrepancies between said evaluated information and said known information,
      stopping iteratively proceeding with said deriving, producing, comparing and modifying when at least one stop criterion is met,

   - at least one output adapted to provide said modified training parameters (722),

   **characterized in that**:

   - said at least one input is adapted to receive said at least one set (71) including clinical feature information (722) related to said embryos,
   - said at least one processor is configured for applying said operations also jointly to said clinical feature information in recursively deriving said at least one step output associated with each of said current images,
   - said known information (713) comprises data on said fertilization outcomes for said respective embryos and said evaluated information comprises predictions on said fertilization outcomes for said respective embryos,
   said device (6) for training being advantageously adapted to a device (1) for predicting according to any of claims **1** to **10.**

13. A method for training a machine learning system configured for predicting an outcome of an in-vitro fertilization of an embryo, said method comprising:

   - receiving (81) at least one set of data representative of time sequences comprising images (711) of embryos during in-vitro developments of said embryos and of known information (713) relevant to fertilization outcomes for said respective embryos;
   - iteratively over said time sequences:

      recursively deriving (83), over respective current images of any of said time sequences, at least one step output (311) associated with one of said current images (210), from operations applied jointly to said one of said current images and to at least one step output associated with at least one of a preceding image and a following image in said any of said time sequences, said deriving relying on training parameters of said machine learning system,

producing (84) evaluated information relevant to said fertilization outcomes for said respective embryos from at least one of said step outputs,

comparing said evaluated information with said known information, and modifying (85) said training parameters so as to reduce discrepancies between said evaluated information and said known information (713),

stopping (86) iteratively proceeding with said deriving, producing, comparing and modifying when at least one stop criterion is met,

- providing (87) said modified training parameters,

**characterized in that** said method comprises:

- receiving (81) said at least one set (71) including clinical feature information (712) related to said embryos,
- applying said operations also jointly to said clinical feature information in recursively deriving said at least one step output associated with each of said current images,

said known information (713) comprising data on said fertilization outcomes for said respective embryos and said evaluated information comprising predictions on said fertilization outcomes for said respective embryos,

said method for training being advantageously executed by a device (6) for training according to claim **12.**

14. A computer program comprising software code adapted to perform at least one of a method for predicting according to any of claims **1** to **10** and a method for training according to claim **13** when said computer program is executed by a processor.

**FIG. 1**

321

**FIG. 2**

322

**FIG.3**

Time frames →

| First frame | | ... | | Last frame | ← 210 |
|---|---|---|---|---|---|
| CNN | CNN | ... | CNN | CNN | ← 131 |
| RNN → | RNN → | ... → | RNN → | RNN | ← 132 |

141 — **Fully connected layer**

310 — **Outcome prediction**

**FIG. 4**

FIG. 5

FIG. 6

**FIG. 7**

21

22

Image data

Clinical features

41

Receiving

42

Preprocessing

43

Recursive time loop

44

Extracting predictions

Outcome
predictions

31

Importance
mapping

32

FIG.8

**FIG. 9**

**FIG. 10**

Training datasets

Initial training parameters

71     721

Receiving — 81

Preprocessing — 82

Recursive time loop — 83

Extracting predictions — 84

Modifying ML parameters — 85

N   86

Stop test assessment

Y

722

Modified training parameters ← Outputting ML parameters — 87

**FIG. 11**

9

91 — CPU

92 — Graphics card

920 — GPUs

921 — GRAM

9211 — Image data

9212 — Extracted features

9213 — Importance mapping

930 — Display

93

94 — I/O devices

96 — ROM

99 — RF unit

97 — RAM

Prog — 970

Image data — 971

Clinical features — 972

Outcome predictions — 973

Importance mapping — 974

ML parameters — 975

95

98 — Power supply

**FIG. 12**

Europäisches
Patentamt

European
Patent Office

Office européen
des brevets

# EUROPEAN SEARCH REPORT

Application Number

EP 20 30 5914

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MANOJ KUMAR KANAKASABAPATHY ET AL: "Deep learning mediated single time-point image-based prediction of embryo developmental outcome at the cleavage stage", ARXIV.ORG, CORNELL UNIVERSITY LIBRARY, 201 OLIN LIBRARY CORNELL UNIVERSITY ITHACA, NY 14853, 21 May 2020 (2020-05-21), XP081677007, * page 3, paragraph 1-3 * * page 2, paragraph 1 * * page 8, paragraph 3.1; figure 3 * | 1-14 | INV. G16H50/20 |
| Y | FADCHAR NEMILYN A ET AL: "Prediction Model for Chicken Egg Fertility Using Artificial Neural Network", 2020 IEEE 7TH INTERNATIONAL CONFERENCE ON INDUSTRIAL ENGINEERING AND APPLICATIONS (ICIEA), IEEE, 16 April 2020 (2020-04-16), pages 916-920, XP033774501, DOI: 10.1109/ICIEA49774.2020.9101966 [retrieved on 2020-05-26] * page 919, column 1, paragraph 1 * * page 917, paragraph II * | 1-14 | |
| Y | US 2015/147770 A1 (RAMSING NIELS B [DK] ET AL) 28 May 2015 (2015-05-28) * paragraphs [0036], [0137], [0121] - [0123] * | 1-14 | TECHNICAL FIELDS SEARCHED (IPC) G16H |

-/--

The present search report has been drawn up for all claims

| Place of search | Date of completion of the search | Examiner |
|---|---|---|
| Munich | 31 December 2020 | Laub, Christoph |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 30 5914

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SILVA-RODRIGUEZ JULIO ET AL: "Predicting the Success of Blastocyst Implantation from Morphokinetic Parameters Estimated through CNNs and Sum of Absolute Differences", 2019 27TH EUROPEAN SIGNAL PROCESSING CONFERENCE (EUSIPCO), EURASIP, 2 September 2019 (2019-09-02), pages 1-5, XP033660122, DOI: 10.23919/EUSIPCO.2019.8902520 [retrieved on 2019-11-15] * the whole document * ----- | 1-14 | |
| A | IWATA K ET AL: "Deep learning based on images of human embryos obtained from high-resolusion time-lapse cinematography for predicting good-quality embryos", FERTILITY AND STERILITY, ELSEVIER, AMSTERDAM, NL, vol. 110, no. 4, 13 September 2018 (2018-09-13), XP085473459, ISSN: 0015-0282, DOI: 10.1016/J.FERTNSTERT.2018.07.615 * the whole document * ----- | 1-14 | |
| A,D | KRAGH MIKKEL F ET AL: "Automatic grading of human blastocysts from time-lapse imaging", COMPUTERS IN BIOLOGY AND MEDICINE, NEW YORK, NY, US, vol. 115, 15 October 2019 (2019-10-15), XP085973989, ISSN: 0010-4825, DOI: 10.1016/J.COMPBIOMED.2019.103494 [retrieved on 2019-10-15] * the whole document * ----- | 1-14 | TECHNICAL FIELDS SEARCHED (IPC) |

The present search report has been drawn up for all claims

| Place of search | Date of completion of the search | Examiner |
|---|---|---|
| Munich | 31 December 2020 | Laub, Christoph |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 30 5914

This annex lists the patent family members relating to the patent documents cited in the above-mentioned European search report.
The members are as contained in the European Patent Office EDP file on
The European Patent Office is in no way liable for these particulars which are merely given for the purpose of information.

31-12-2020

| Patent document cited in search report | Publication date | Patent family member(s) | | Publication date |
|---|---|---|---|---|
| US 2015147770 A1 | 28-05-2015 | AU | 2013269608 A1 | 04-12-2014 |
| | | CN | 104508123 A | 08-04-2015 |
| | | EP | 2855664 A1 | 08-04-2015 |
| | | US | 2015147770 A1 | 28-05-2015 |
| | | WO | 2013178785 A1 | 05-12-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020001914 A, Vitrolife
- WO 2019113643 A, Virtus
- WO 2019068073 A

### Non-patent literature cited in the description

- **M. F. KRAGH et al.** Automatic grading of human blastocysts from time-lapse imaging. *Computers in Biology and Medicine,* 2019, 115-103494
- **D. TRAN et al.** Deep learning as a predictive tool for fetal heart pregnancy following time-lapse incubation and blastocyst transfer. *Human Reproduction,* 2018, 1-8
- **O. RONNEBERGER et al.** U-Net: Convolutional Networks for Biomedical Image Segmentation. *MICCAI,* 2015, vol. 9351, 234-241
- **S. M. LUNDBERG ; S.-I. LEE.** A Unified Approach to Interpreting Model Predictions. *31st Conf. on Neural Information Processing Systems, Long Beach,* 2017
- **R. R. SELVARAJU et al.** Grad-CAM Visual Explanations from Deep Networks via Gradient-based Localization. *IEEE ICCV,* 2017, 618-626